# FASCICULE DE BREVET EUROPEEN

(11) **EP 3 357 517 B1**
(45) Date de publication et mention de la délivrance du brevet: **10.11.2021**
(21) Numéro de dépôt: 18154599.7
(22) Date de dépôt: 01.02.2018
(51) Int. Cl.: A61L 24/00

(54) **COMPOSITION ADHESIVE ET ELEMENT DE FIXATION SUR LA PEAU HUMAINE**
KLEBSTOFFZUSAMMENSETZUNG UND BEFESTIGUNGSELEMENT FÜR DIE MENSCHLICHE HAUT
ADHESIVE COMPOSITION AND FIXATION ELEMENT FOR HUMAN SKIN

(30) Priorité: 03.02.2017 FR 1750958
(43) Date de publication de la demande: 08.08.2018
(73) Titulaire: B.Braun Medical SAS, 92100 Boulogne-Billancourt (FR)
(72) Inventeur: Delphine, Burlot, 64600 Anglet (FR); Lassalle, Paul, 64600 Anglet (FR); Lalet, Laurent, 64600 Anglet (FR)
(74) Mandataire: August Debouzy

(56) Documents cités:
- WO-A1-2011/157278
- US-A- 4 855 335

## Description

### Domaine de l'invention

L'invention concerne une composition adhésive destinée essentiellement à constituer un élément adhésif par exemple pour poches de recueil de fluides corporels, telles que des poches de stomie ou des éléments analogues tels que des pansements.

### Arrière-plan technique

On connaît des compositions adhésives, notamment pour poche de recueil de fluides corporels, qui comportent une "phase continue" à base de polymères, élastomères, etc. et une "phase discontinue" contenant essentiellement des hydrocolloides.

La phase continue contient différents copolymères, polymères, élastomères qui ont une fonction de création d'une texture, éventuellement des plastifiants qui ont une fonction d'augmentation de souplesse de la composition, et des résines d'adhésivité, parfois désignées par le néologisme "tackifiant" qui correspond au terme anglais "tackifier".

Cette terminologie, qui distingue les ingrédients de texture (élastomères, polymères, copolymères) des plastifiants et des agents d'adhésivité ou résines tackifiantes est à considérer dans le domaine considéré et n'est pas nécessairement absolue. En effet, la plupart des polymères, copolymères et élastomères ont plus ou moins des propriétés de texture (à moins qu'ils ne soient liquides ou huileux), de plastifiant et d'agent d'adhésivité par exemple en présence d'autres ingrédients, ou certaines résines tackifiantes peuvent aussi avoir des fonctions de plastifiant. Il faut donc considérer l'action de ces ingrédients dans le cadre des compositions adhésives utilisées pour la fixation, directe ou indirecte, des poches de recueil de fluides corporels. Cependant, cette terminologie permet de distinguer les différentes compositions adhésives utilisées pour la fixation des poches de recueil de fluides corporels.

Les compositions adhésives pour poches de recueil de fluides corporels sont utilisées pour la fixation d'une poche par collage direct à la peau d'un être humain, autour d'un orifice d'évacuation d'excréments. Le collage peut être prévu pour durer un temps relativement court, qui peut être inférieur à une journée, ou un temps relativement important, qui peut être de l'ordre d'une semaine. Les compositions adhésives utilisées pour ces diverses applications ont donc des propriétés différentes, selon qu'elles doivent rester fixées pendant un temps plus ou moins long. Cependant, elles ont toutes un certain nombre de propriétés communes.

Les compositions adhésives de fixation des poches de recueil de fluides corporels doivent d'abord posséder certaines propriétés mécaniques. Ces propriétés mécaniques sont essentiellement d'abord la tenue mécanique d'une poche, qui peut avoir un certain poids, sans décollement de la composition adhésive de la peau, ensuite l'absence de fuite au niveau de la composition adhésive, et enfin une bonne adaptabilité destinée à donner un bon confort qui est non seulement une bonne commodité à la mise en place et à l'enlèvement, mais surtout une souplesse suffisante pour que le patient oublie autant que possible la présence de la composition adhésive et de la poche.

Les compositions adhésives de fixation des poches de recueil de fluides corporels doivent ensuite posséder certaines propriétés chimiques. Il faut essentiellement que la composition adhésive ne provoque pas une irritation cutanée.

On connaît de telles poches depuis de nombreuses années. Typiquement ces poches contiennent à titre de polymères des polymères du type SIS ou SBS, éventuellement en mélange avec des polymères du type PIB ou élastomère butyle. Cependant, de telles associations de polymères ne sont pas parfaites. Notamment, si l'addition de PIB est bénéfique elle conduit à une certaine décohésion. Il est donc souhaitable de disposer d'une formulation de polymères qui permette l'obtention d'une composition qui a un« tack »important pour pouvoir s'afficher rapidement sur la peau, adhère fortement en se conformant sur le relief abdominal pour supporter le poids et les contraintes d'une poche, et a assez de cohésion pour se retirer sans difficultés et sans résidus.

Le document US4855335 décrit une composition comprenant 12% à 20% d'huile minérale, 30% à 40% d'une résine tackifiante, 15% à 30% d'un hydrocolloïde, 5% à 10% d'un ou plusieurs polyisobutylenes, 7% à 15% d'un copolymètre styrènique, et 1% à 8% d'un copolymère éthylène acétate de vinyle. Il est indiqué que l'EVA augmente la stabilité dimensionnelle.

Cependant cette formulation n'est pas satisfaisante, les quantités notamment de résine et/ou hydrocolloïde n'étant pas adaptées pour les compositions actuelles.

Le document WO2011/157278 décrit des compositions adhésives comprenant 10-50% d'une phase polaire, cette phase polaire comprenant un EVA et une huile polaire, 10-50% d'une phase apolaire, cette phase apolaire comprenant un polymère bloc de styrène, et un polyisobutylène ou un élastomère butyle, et jusqu'à 60% d'un hydrocolloïde.

Cependant cette formulation n'est pas satisfaisante, la présence d'une phase polaire et notamment d'une huile polaire imposant des contraintes en termes de composants de la composition.

L'invention permet donc d'obtenir le compromis recherché sans les contraintes de l'état de la technique.

### Résumé de l'invention

L'invention a pour objet une composition adhésive destinée à assurer la fixation sur la peau humaine, comprenant une phase continue et une phase discontinue d'hydrocolloïdes, la phase continue comprenant en poids, sur la base du poids total de la composition adhésive :
(i) 1 à 12% en poids d'un polymère séquencé de type styrène-isoprène-styrène ou styrène-butadiène-styrène,
(ii) (a) 1 à 15% d'un polymère de type élastomère butyl, éventuellement en mélange avec jusqu'à 25% en poids de d'un polymère de type polyisobutylène, ou
(iii) 1 à moins de 10% d'un polymère de type éthylène vinyl acétate,
la somme de ces trois types de polymères représentant de 10 à 40%.

Selon l'invention, la composition est sensiblement exempte d'huile polaire et/ou dans laquelle phase polaire représente moins de 10% en poids.

Selon un autre mode de réalisation, la phase continue comprend :
(i) 2 à 8% en poids d'un polymère séquencé de type styrène-isoprène-styrène ou styrène-butadiène-styrène,
(ii) (a) 2 à 10% d'un polymère de type élastomère butyl, éventuellement en mélange avec jusqu'à 25% en poids de polymère de type polyisobutylène,
(iii) 2 à moins de 10% d'un polymère éthylène vinyl acétate.

Selon un autre mode de réalisation, la phase continue comprend :
(ii) (a) 2 à 10% d'un polymère de type élastomère butyl, exempt de polymère de type polyisobutylène.

Selon un autre mode de réalisation, la phase continue comprend :
(ii) (a) 2 à 10% d'un polymère de type élastomère butyl, en mélange avec de 10 à 25% en poids de polymère de type polyisobutylène.

Selon un autre mode de réalisation, le polymère éthylène vinyl acétate comprend de 30 à 70% en masse du monomère acétate de vinyle.

Selon un autre mode de réalisation, la composition comprend 10 à 30% de résine tackifiante.

Selon un autre mode de réalisation, la composition comprend 40 à 60% de phase discontinue d'hydrocolloïdes.

Selon un autre mode de réalisation, la phase discontinue comprend une majorité d'au moins un composé choisi parmi les fibres de cellulose, la carboxyméthylcellulose, sodique, réticulée ou autre, et l'hydroxyéthylcellulose, ainsi que des composés analogues à la gomme tels que gomme guar ou karaya ou arabique, et des substances telles que les xanthanes, les amidons par exemple amidon de pomme de terre, les alginates, la pectine, la gélatine, le psyllium, l'extrait de caroube, l'agarose, les carraghénines les polyacrylamides, ou leurs mélanges.

Selon un autre mode de réalisation, la phase discontinue est exempte de produit d'origine animale.

L'invention a aussi pour objet un élément de fixation de poche de recueil de fluides corporels, comprenant la composition adhésive selon l'invention et, sur la face opposée à celle qui est destinée à être au contact de la peau, un film.

Selon un autre mode de réalisation, le film est un film de polyéthylène ou de copolymère ethylène-acétate de vinyle ou leur mélange, de préférence avec une épaisseur de 25 à 100µm.

L'invention fournit donc une composition adhésive, savoir une composition adhésive à la pression.

L'utilisation des trois composés, SIS ou SBS, EVA et élastomère butyl, éventuellement en mélange avec du PIB dans la masse polymère selon les proportions indiquées permet d'obtenir le compromis et les trois comportements complémentaires. Le copolymère styrénique (SIS ou SBS) permet d'obtenir un adhésif avec du tack immédiat important, une cohésion et une structure dans la composition adhésive. L'EVA permet de diminuer l'élasticité du réseau en le rendant plus plastique. Le polyisobutylène (PIB) ou Butyl permet de prendre le relai des résines en donnant une adhésivité plus forte, notamment avec des bas poids moléculaires en augmentant la conformabilité de la composition adhésive.

Dans la présente demande, tous les % sont donnés en poids par rapport au poids total de la composition adhésive.

### Description de modes de réalisations de l'invention

### Polymères de type SIS ou SBS

Ces polymères sont classiques et connus dans la technique. Ce sont des copolymères blocs à base de styrène et isoprène ou butadiène (ou un mélange des deux).

Le copolymère de texture de la phase continue de type styrène-isoprène-styrène ou styrène-butadiène-styrène, est constitué de préférence d'un copolymère séquence de styrène-isoprène-styrène. Ces polymères peuvent contenir des quantités variables de diblocs ou triblocs, de séquences doubles ou triples.

On pourra se référer pour une description de ces polymères au document WO2011/157278, de la page 8, lignes 6-18, passage auquel il est expressément renvoyé. On pourra aussi se référer au document US4855335, de la col.3, lignes 16-23 passage auquel il est expressément renvoyé.

Des exemples de copolymères de type styrène-isoprène-styrène ou styrène-butadiène-styrène texture convenant selon l'invention sont par exemple certains copolymères "Kraton" de Shell Chemical Company, par exemple de type D11 (D1161, D1117 et D1112), ou encore ces copolymères tels que "Vector 4113" de Dexco ou "Cariflex S-1707" de Shell.

### Polymères de type butyl ou PIB

Ces polymères sont aussi classiques.

Le polymère classiquement identifié sous le terme de « butyl » est un élastomère polybutylique comprenant une partie minoritaire d'isoprène. On pourra utiliser ceux commercialisés sous les dénominations Butyl 268 de chez Exxon, ou Butyl BK de chez Togliatti / Nizhnekamsk.

Le poids moléculaire de l'élastomère butyl est adapté à l'application, par exemple un poids moléculaire Mn de 200000 à 600000.

Le polymère PIB est aussi bien connu de l'homme du métier. On pourra utiliser ceux de la gamme Oppanol disponible chez BASF, avec par exemple les grades B10, B12 et B15. Le poids moléculaire du PIB est adapté à l'application, par exemple un poids moléculaire Mn inférieur 100000, de préférence de 40000 à 60000.

### EVA

Ce type de polymère est aussi connu. On pourra par exemple utiliser ceux comprenant de 30 à 70% en poids de monomère vinyl acétate.

On peut utiliser ceux de la gamme LEVAMELT des grades 400 ou 700 ou de la gamme Evatane.

### Résines tackifiantes

Les résines tackifiantes utilisées dans l'invention sont classiques.

Plus précisément, les résines tackifiantes l'agent d'adhésivité utilisé peuvent comprendre un mélange de résines tackifiantes ayant une forte activité, par exemple à base de résines terpéniques, et/ou ayant une activité moyenne, et/ou ayant une activité faible mais présentant en outre des propriétés renforçantes. On pourra se référer au document EP1871845 pour une description des tests appropriés.

Ces résines tackifiantes sont classiques. On peut ainsi utiliser des résines du type terpéniques, par ex. terpène-styrène, pouvant avoir une température de ramollissement d'environ 105°C (méthode à bille et anneau) et une masse moléculaire en masse de l'ordre de 1000. On peut aussi utiliser des résines ayant aussi une action renforçante. Par exemple on peut utiliser un polymère thermoplastique dérivé essentiellement de α-méthylstyrène ayant par exemple une masse moléculaire en masse comprise entre 300 et 3000. On peut ainsi moduler l'élasticité de la composition. On peut encore utiliser des résines totalement hydrogénées.

Des exemples de telles résines sont les résines disponibles dans le commerce sous les dénominations Regalite, Arkon, Dercolyte, Piccolyte S, Zonatac, Kristalex, Piccotex et Escorez.

Les quantités respectives des différents types de résines peuvent varier comme l'appréciera l'homme du métier.

### Hydrocolloïde

La phase discontinue constituée de polymères hydrophiles sous forme d'hydrocolloïdes est du type bien connu dans la technique, contenant une quantité importante de composés tels que les fibres de cellulose, la carboxyméthylcellulose, sodique, réticulée ou autre, et l' hydroxyéthylcellulose , ainsi que des composés analogues à la gomme guar, et des substances telles que les xanthanes, les alginates , la pectine, la gélatine, le psyllium, l'extrait de caroube, la gomme arabique, l'agarose, les carraghénines et des polyacrylamides.

Les hydrocolloïdes utilisés dans l'invention sont classiques.

### Plastifiant

On utilisera un plastifiant, qui de préférence est une huile apolaire. Un exemple d'une telle huile ou plastifiant est une polyalphaoléfine, comme celle disponible sous la dénominatio, Durasyn. On peut aussi utiliser une huile minérale, une paraffine, une huile de ricin, une cire de paraffine de synthèse de Shell, la résine "Sun 5512" de Sun ou "Primol" de Hercules.

La composition adhésive peut comporter d'autres agents ou additifs, comme un agent à action médicinale, par exemple choisi parmi le chitosane et le α-L-fucose, ou des additifs comme les antioxydants.

L'invention concerne aussi un élément de fixation de poche de recueil de fluides corporels comprenant la composition adhésive précitée et, sur la face opposée à celle qui doit être au contact de la peau, un film de revêtement classique connu dans la technique.

Un tel film est un film de polyéthylène ou EVA, d'épaisseur adaptée.

### Exemples de composition

On prépare les compositions du tableau par mélange des constituants, selon les proportions en poids. Les composants sont donnés dans le tableau. L'ordre d'introduction est celui qui apparaît dans le tableau.

Après chaque introduction, un malaxage d'environ 10 min est mis en œuvre pour obtenir une bonne homogénéité. Le malaxage final dure environ 50 min. La composition ainsi obtenue est extrudée, avec un temps d'extrusion d'environ 60 min, sous forme d'une feuille de 1 mm d'épaisseur et découpée en éléments utilisés pour des essais de fixation de poches de recueil de fluides corporels.

Les éléments sont ensuite testés.

Fmax et Tack: Le test consiste en l'arrachement d'un poinçon exécuté à l'aide d'une machine de traction et la mesure de la force nécessaire à l'arrachement du poinçon (Fmax), dans des conditions déterminées de temps de contact avec la composition avant arrachement et de vitesse d'arrachement. Le tack est mesuré avec le même test mais avec des temps de contact très courts.

Fcompression et % relaxation : le test consiste en un test de compression exécuté à l'aide d'une machine de traction pour mesurer la rigidité (Fcompression) ainsi que la capacité de relaxation (% relaxation), dans des conditions déterminées de temps de contact avec la composition.

Le tableau qui suit donne les compositions et les valeurs des mesures des tests.

| **Formules** | | **1** | **2** | **3** | **4** | **5** | **6** |
|---|---|---|---|---|---|---|---|
| SIS | Kraton D1161 | 3,04 | | 6,50 | | | 4,56 |
| | Vector 4113 | | 3,04 | | 6,5 | 5,5 | |
| EVA | Levamelt 686 | 3 | 3 | 2,6 | 2,6 | 6,5 | 3 |
| | Levamelt 400/ EVATAN 40/55 | | | 2,1 | 2,1 | 3 | 2 |
| Butyl | Butyl 268 | 7,44 | 7,44 | 7,27 | 7,27 | 3 | 3 |
| PIB | Oppanol B15 | | | | | 13 | 12 |
| | Oppanol B12 | 11,00 | 11 | | | | 6 |
| | Oppanol B10 | 9 | 9 | | | | |
| Résines | Régalite | 9,02 | | | | 3,3 | |
| | Dercolyte | | | 15,43 | | | |
| | Piccolyte S | | 5,3 | | 13,33 | 7,3 | 7,7 |
| | Kristalex | | 3,46 | 6,5 | | | |
| | Piccotex | 3,46 | | | 6,5 | 5,5 | 5,19 |
| | Escorez | | 3,72 | | | | |
| Plastifiant | Durasyn | 5,04 | 5,04 | 11,6 | 13,7 | 9,9 | 7,55 |
| Hydrocolloïdes | CMC7M | 12,00 | 8 | 11 | | | |
| | Guar | 10,00 | 9 | 9 | 12 | 7 | 11 |
| | CMC12M | | 7 | | 14 | 15 | 14 |
| | Gélatine | 16,00 | 14 | 12 | 11 | | 15 |
| | Pectine | 11,00 | 11 | 11 | 11 | 12 | 9 |
| | Superabsorbant A500 | | | 5 | | 8,5 | |

| | | | | | | | |
|---|---|---|---|---|---|---|---|
| Caractéristiques | Tack | 1,15 | 1,15 | 1,27 | 1,25 | 1,35 | 1,20 |
| | Fmax | 4,60 | 4,80 | 3,20 | 3,40 | 3,80 | 3,00 |
| | F compression | 3,10 | 3,00 | 2,50 | 2,40 | 2,60 | 2,50 |
| | % relaxation | 67,00 | 65,00 | 60,00 | 60,00 | 72,00 | 65,00 |

On considère maintenant d'autres propriétés des compositions selon l'invention.

### Essais chez les patients

Des essais ont été réalisés dans des conditions de milieu hospitalier, c'est-à-dire dans lesquelles la pose et l'enlèvement sont effectués par du personnel hospitalier. Le personnel hospitalier ou les patients ont déterminé plusieurs paramètres parmi lesquels l'adhésivité immédiate, la présence de résidus sur la peau après le retrait de la composition, la facilité de l'application de la poche, la facilité de retrait de la poche, la conformabilité et l'adhésivité de la poche lorsqu'elle est portée par le patient.

Les résultats obtenus sont excellents et le compromis est atteint.

Ainsi, l'invention concerne des compositions adhésives et des éléments de fixation qui présentent le compromis recherché pour les poches de stomie.

## Revendications

1. Composition adhésive destinée à assurer la fixation sur la peau humaine, comprenant une phase continue et une phase discontinue d'hydrocolloïdes, la phase continue comprenant en poids, sur la base du poids total de la composition adhésive :
(i) 1 à 12% en poids d'un polymère séquencé de type styrène-isoprène-styrène ou styrène-butadiène-styrène,
(ii) (a) 1 à 15% d'un polymère de type élastomère butyl, éventuellement en mélange avec jusqu'à 25% en poids de d'un polymère de type polyisobutylène,
(iii) 1 à moins de 10% d'un polymère de type éthylène vinyl acétate,
la somme de ces trois types de polymères représentant de 10 à 40%,
ladite composition étant sensiblement exempte d'huile polaire et/ou dans laquelle la phase polaire représentant moins de 10% en poids.

2. Composition selon la revendication 1, dans laquelle la phase continue comprend :
(i) 2 à 8% en poids d'un polymère séquencé de type styrène-isoprène-styrène ou styrène-butadiène-styrène,
(ii) (a) 2 à 10% d'un polymère de type élastomère butyl, éventuellement en mélange avec jusqu'à 25% en poids de polymère de type polyisobutylène,
(iii) 2 à moins de 10% d'un polymère éthylène vinyl acétate.

3. Composition selon la revendication 1 ou 2, comprenant :
(ii) (a) 2 à 10% d'un polymère de type élastomère butyl, exempt de polymère de type polyisobutylène.

4. Composition selon la revendication 1 ou 2, comprenant :
(ii) (a) 2 à 10% d'un polymère de type élastomère butyl, en mélange avec de 10 à 25% en poids de polymère de type polyisobutylène.

5. Composition selon l'une quelconque des revendications précédentes, dans laquelle le polymère éthylène vinyl acétate comprend de 30 à 70% en masse du monomère acétate de vinyle.

6. Composition selon l'une des revendications 1 à 5, comprenant 10 à 30% de résine tackifiante.

7. Composition selon l'une des revendications 1 à 6, comprenant 40 à 60% de phase discontinue d'hydrocolloïdes.

8. Composition selon l'une des revendications 1 à 7, dans laquelle la phase discontinue comprend une majorité d'au moins un composé choisi parmi les fibres de cellulose, la carboxyméthylcellulose, sodique, réticulée ou autre, et l'hydroxyéthylcellulose, ainsi que des composés analogues à la gomme tels que gomme guar ou karaya ou arabique, et des substances telles que les xanthanes, les amidons par exemple amidon de pomme de terre, les alginates, la pectine, la gélatine, le psyllium, l'extrait de caroube, l'agarose, les carraghénines les polyacrylamides, ou leurs mélanges.

9. Composition selon l'une des revendications 1 à 8, dans laquelle la phase discontinue est exempte de produit d'origine animale.

10. Elément de fixation de poche de recueil de fluides corporels, comprenant la composition adhésive selon l'une quelconque des revendications précédentes et, sur la face opposée à celle qui est destinée à être au contact de la peau, un film.

11. Elément selon la revendication 10, dans lequel le film est un film de polyéthylène ou de copolymère ethylène-acétate de vinyle ou leur mélange, de préférence avec une épaisseur de 25 à 100µm.

## Patentansprüche

1. Klebstoffzusammensetzung, die ausgelegt ist, um die Befestigung auf der menschlichen Haut sicherzustellen, umfassend eine kontinuierliche Phase und eine diskontinuierliche Phase von Hydrokolloiden, wobei die kontinuierliche Phase basierend auf dem Gesamtgewicht der Klebstoffzusammensetzung Folgendes nach Gewicht umfasst:
(i) 1 bis 12 Gew% eines sequenzierten Polymers vom Typ Styrol-Isopren-Styrol oder Styrol-Butadien-Styrol,
(ii) (a) 1 bis 15 % eines Polymers vom Typ Butylelastomer, eventuell als Gemisch mit bis zu 25 Gew% eines Polymers vom Typ Polyisobutylen,
(iii) 1 bis weniger als 10 % eines Polymers vom Typ Ethylenvinylacetat, wobei die Summe dieser drei Typen von Polymeren von 10 bis 40 % aufweist, wobei die Zusammensetzung im Wesentlichen frei von polarem Öl ist, und/oder wobei die polare Phase weniger als 10 Gew% darstellt.

2. Zusammensetzung nach Anspruch 1, wobei die kontinuierliche Phase Folgendes umfasst:
(i) 2 bis 8 Gew% eines sequenzierten Polymers vom Typ Styrol-Isopren-Styrol oder Styrol-Butadien-Styrol,
(ii) (a) 2 bis 10 % eines Polymers vom Typ Butylelastomer, eventuell als Gemisch mit bis zu 25 Gew% Polymer vom Typ Polyisobutylen,
(iii) 2 bis weniger als 10 % eines Ethylenvinylacetatpolymers.

3. Zusammensetzung nach Anspruch 1 oder 2, umfassend
(ii) (a) 2 bis 10 % eines Polymers vom Typ Butylelastomer, frei von Polymer vom Typ Polyisobutylen.

4. Zusammensetzung nach Anspruch 1 oder 2, umfassend:
(ii) (a) 2 bis 10 % eines Polymers vom Typ Butylelastomer, als Gemisch mit 10 bis 25 Gew% Polymer vom Typ Polyisobutylen.

5. Zusammensetzung nach einem der vorhergehenden Ansprüche, wobei das Ethylenvinylacetatpolymer 30 bis 70 Massenprozent des Vinylacetatmonomers umfasst.

6. Zusammensetzung nach einem der Ansprüche 1 bis 5, umfassend 10 bis 30 % klebrigmachendes Harz.

7. Zusammensetzung nach einem der Ansprüche 1 bis 6, umfassend 40 bis 60 % diskontinuierliche Phase von Hydrokolloiden.

8. Zusammensetzung nach einem der Ansprüche 1 bis 7, wobei die diskontinuierliche Phase zum großen Teil mindestens eine Verbindung umfasst, ausgewählt aus Zellulosefasern, Natrium-, retikulierter oder anderer Carboxymethylzellulose und Hydroxyethylzellulose, sowie analoge Verbindungen mit Gummi wie z. B. Guargummi oder Karayagummi oder Gummiarabikum und Substanzen wie z. B. Xanthane, Stärken wie z. B. Kartoffelstärke, Alginate, Pektin, Gelatine, Psyllium, Johannisbrotextrakt, Agarose, Carrageenane, Polyacrylamide oder ihre Gemische.

9. Zusammensetzung nach einem der Ansprüche 1 bis 8, wobei die diskontinuierliche Phase frei von Produkten tierischen Ursprungs ist.

10. Element zur Befestigung eines Rückhaltebeutels von Körperflüssigkeiten, umfassend die Klebstoffzusammensetzung nach einem der vorhergehenden Ansprüche und, auf der gegenüberliegenden Seite derselben, die ausgelegt ist, um mit der Haut in Kontakt zu sein, einen Film.

11. Element nach Anspruch 10, wobei der Film ein Film aus Polyethylen oder Ethylen-Vinylacetat-Copolymer oder ihrem Gemisch ist, vorzugsweise mit einer Dicke von 25 bis 100 µm.

## Claims

1. Adhesive composition intended to provide the attaching on human skin, comprising a continuous phase and a discontinuous phase of hydrocolloids, the continuous phase comprising by weight, based on the total weight of the adhesive composition:
(i) 1 to 12% by weight of a sequenced polymer of the styrene-isoprene-styrene or styrene-butadiene-styrene type,
(ii) (a) 1 to 15% of a polymer of the butyl elastomer type, optionally in a mixture with up to 25% by weight of a polymer of the polyisobutylene type,
(iii) 1 to less than 10% of a polymer of the ethylene vinyl acetate type,
with the sum of these three types of polymers representing from 10 to 40%,
said composition being substantially free of polar oil and/or wherein polar phase represents less than 10% by weight.

2. Composition according to claim 1, wherein the continuous phase comprises:
(i) 2 to 8% by weight of a sequenced polymer of the styrene-isoprene-styrene or styrene-butadiene-styrene type,
(ii) (a) 2 to 10% of a polymer of the butyl elastomer type, optionally in a mixture with up to 25% by weight of polymer of the polyisobutylene type,
(iii) 2 to less than 10% of an ethylene vinyl acetate polymer.

3. Composition according to claim 1 or 2, comprising:
(ii) (a) 2 to 10% of a polymer of the butyl elastomer type, free of polymer of the polyisobutylene type.

4. Composition according to claim 1 or 2, comprising:
(ii) (a) 2 to 10% of a polymer of the butyl elastomer type, in a mixture with from 10 to 25% by weight of polymer of the polyisobutylene type.

5. Composition according to any of the preceding claims, wherein the ethylene vinyl acetate polymer comprises from 30 to 70% by weight of the vinyl acetate monomer.

6. Composition according to one of claims 1 to 5, comprising 10 to 30% of tackifier resin.

7. Composition according to one of claims 1 to 6, comprising 40 to 60% of discontinuous phase of hydrocolloids.

8. Composition according to one of claims 1 to 7, wherein the discontinuous phase comprises a majority of at least one compound chosen from cellulose fibres, carboxymethylcellulose, sodium, cross-linked or other, and hydroxyethylcellulose, as well as compounds similar to gum such as guar or karaya gum or gum arabic, and substances such as xanthanes, starches for example potato starch, alginates, pectin, gelatine, psyllium, carob extract, agarose, carrageenans, polyacrylamides, or mixtures thereof.

9. Composition according to one of claims 1 to 8, wherein the discontinuous phase is free from products of animal origin.

10. Element for attaching a bag for collecting body fluids, comprising the adhesive composition as claimed in any preceding claim and, on the face opposite that which is intended to be in contact with the skin, a film.

11. Element according to claim 10, wherein the film is a polyethylene film or ethylene-vinyl acetate copolymer or the mixture thereof, more preferably with a thickness of 25 to 100 µm.
